# EUROPEAN PATENT APPLICATION

(11) **EP 4 652 985 A1**
(43) Date of publication of application: **26.11.2025**
(21) Application number: 23917053.3
(22) Date of filing: 14.09.2023
(51) Int. Cl.: A61K 8/46, A61Q 5/02, A61Q 19/10

(54) **CLEANING COMPOSITION CONTAINING SURFACTANT SYSTEM, AND USE THEREOF**

(30) Priority: 18.01.2023 CN 202310060272
(71) Applicant: Zhangjiagang Great Chemical Co., Ltd., Suzhou, Jiangsu 215613 (CN)
(72) Inventor: HUANG, Yongqian, Suzhou, Jiangsu 215613 (CN); ZHU, Hongjun, Suzhou, Jiangsu 215613 (CN)
(74) Representative: Winter, Brandl - Partnerschaft mbB
(86) International application number: PCT/CN2023/118857
(87) International publication number: WO 2024/152585

(57) **Abstract**

A cleaning composition containing a surfactant system, and the use thereof, which relate to the technical field of cleaning compositions and the preparation therefor. The cleaning composition comprises A) an alkanoyl taurate and B) a methyl alkanoyl taurate. By controlling the mass proportion of the alkanoyl taurate to the methyl alkanoyl taurate during an application process, the application of the prepared cleaning composition to a personal care detergent can overcome respective defects of the two raw materials, and exert the respective advantages and characteristics of the two raw materials, such that an obtained washing and caring product is easier to thicken, has a high transparency and good stability, and has relatively good foamability and conditioning property.

## Description

The present disclosure claims priority to Chinese Patent Application No. 202310060272.X, filed with the Chinese Patent Office on January 18, 2023, and entitled "Cleaning Composition Containing Surfactant System and Its Application", the entire contents of which are incorporated by reference in the application.

### TECHNICAL FIELD

The present application relates to a cleaning composition, in particular to a cleaning composition containing a surfactant system and its application.

### BACKGROUND

Surfactants are the most important materials in personal washing care products. With the improvement of living standards, people's awareness of health and environmental protection is constantly increasing, so more and more consumers and businesses will pay attention to environmentally friendly surfactants, especially amino acid surfactants, where alkanoyl taurine salt is one of the best amino acid surfactants.

It is worth mentioning that the correct name for the "taurine surfactant" that has been mass-produced on the market for a long time is methyl alkanoyl taurine salt. Since there has been no commercially mass-produced surfactant of alkanoyl taurine salt for sale and use, methyl alkanoyl taurine salt is often referred to as alkanoyl taurine salt. In fact, although these two surfactants both contain the structural group of taurine, they are significantly different in terms of the raw materials for synthesis and the properties of products, making them two completely different surfactants. The application of methyl alkanoyl taurine salt has become very common, but due to certain shortcomings in its service performance, such as difficulty in thickening, poor compatibility with cations, and instability, there have been few products that have become the main surfactants.

For example, as disclosed in Chinese Patent Publication No. CN110177539A, a bar soap composition is prepared from the following components: at least one cleaning agent selected from soap and a first surfactant; and at least one taurine salt surfactant selected from salts of fatty acid amide of taurine, salts of fatty acid amide of N-methyl taurine, and combinations thereof. The taurine salt surfactant is different from the first surfactant. The composition provided by this application is in the form of a solid bar. This application also discloses other personal care and household care products containing taurine salt surfactants, and further defines that the taurine salt surfactants therein are sodium methyl oleoyl taurate. In addition, this application mainly focuses on the research of the composition to alleviate skin irritation, skin inflammation, and/or improve the cellular repair function of the skin, and does not involve the stability of the composition or the combability of products.

In the course of research, the inventors of the present application unexpectedly found that when methyl alkanoyl taurine salt and alkanoyl taurine salt are mixed and used in combination, the respective disadvantages of the two raw materials can be overcome, and their respective advantages and characteristics can be brought into play. The resulting cleaning product is easier to thicken, high in transparency and good in stability, and has better foaming power and conditioning property.

Chinese Patent Publication No. 202080033788.X discloses a layered liquid composition, which contains acyl hydroxyethyl sulfonate, methyl acyl taurate, and an amphoteric and/or zwitterionic surfactant. Unexpectedly, applicants have discovered that enhanced lather is achieved when the ratio of hydroxyethyl sulfonate to taurate is maintained at about 1:1.

However, it should be noted that the alkanoyl taurine salt prepared in the prior art has the problem of too high Krafft point, and even a product containing 30% of the alkanoyl taurine salt will form a hard block at room temperature, making it inconvenient to use. Furthermore, although it can be easily thickened and easily made into a transparent and stable product, there is also a phenomenon that the product is precipitated and unstable in winter.

Therefore, it is necessary to provide a cleaning composition that are not prone to agglomeration even if used in winter, and a preparation method thereof.

### SUMMARY

Based on the problems existing in the prior art, the present application provides a cleaning composition containing a surfactant system and its application. In the implementation process, it is unexpectedly found that when two taurine surfactants are used together, that is, alkanoyl taurine salt and methyl alkanoyl taurine salt are used together, the disadvantages of the two raw materials can be overcome and their respective advantages are leveraged.

In order to achieve the above object, the present application adopts the following technical solutions:

In one aspect, the present application provides a cleaning composition containing a surfactant system, including the following components:
A) alkanoyl taurine salt, the structural formula of the alkanoyl taurine salt being as shown in formula 1 below: 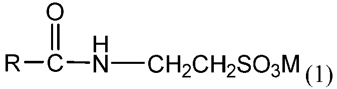 where R is a saturated or unsaturated hydrocarbon group of C7-C21, the saturated or unsaturated hydrocarbon group contains branched or unbranched chains, and M is an alkali metal ion or an alkaline earth metal ion; and
B) methyl alkanoyl taurine salt, the structural formula of the methyl alkanoyl taurine salt being as shown in formula 2 below: 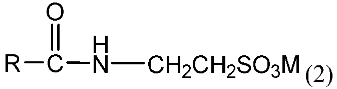 where R is a saturated or unsaturated hydrocarbon group of C7-C21, the saturated or unsaturated hydrocarbon group contains branched or unbranched chains, and M is an alkali metal ion or an alkaline earth metal ion.

Preferably, the C7-C21 are single carbon chains or mixed carbon chains.

More preferably, each of the C7-C21 is one of lauryl, myristyl or cocoyl.

Preferably, the alkali metal ion or alkaline earth metal ion is selected from one of sodium, potassium, calcium, and magnesium.

More preferably, the alkali metal ion or alkaline earth metal ion is selected from one of sodium, potassium, and calcium.

Further preferably, the alkali metal ion or alkaline earth metal ion is selected from one of sodium or potassium.

As some preferred embodiments, the total amount of A and B in the cleaning composition is not less than 0.5wt%, preferably 3-30wt%.

As some preferred embodiments, the weight ratio of A and B in the cleaning composition is within a range of 10:1 to 1:10.

Preferably, the weight ratio of A and B in the cleaning composition is within a range of 7:3 to 4:6.

As some preferred embodiments, the cleaning composition further includes a surfactant C besides A and B; and the surfactant C is selected from one or more of anionic surfactants, cationic conditioning agents, non-ionic surfactants, and zwitterionic surfactants.

The anionic surfactants include, but are not limited to, alkyl sulfates, alkyl ether sulfates, alkyl sulfonates, alkaryl sulfonates, alpha-olefin sulfonates, alkyl amide sulfonates, alkaryl polyether sulfates, alkyl acylamino ether sulfates, alkyl monoglyceryl ether sulfates, alkyl monoglyceride sulfates, alkyl monoglyceride sulfonates, alkyl succinates, alkyl sulfosuccinates, alkyl sulfosuccinates, alkyl ether thiosuccinates, alkyl acylamino sulfosuccinates, alkyl thioacetates, alkyl phosphates, alkyl ether phosphates, alkyl ether carboxylates, alkyl acylamino ether carboxylates, N-alkyl amino acids, N-acyl amino acids, alkyl peptides, alkyl hydroxypropyl sulfonates, alkyl hydroxyethyl sulfonates, carboxylates, and the like. The acyl groups are derived from fatty acids; the cationic parts of the aforementioned salts are selected from sodium, potassium, magnesium, ammonium, monoethanolamine, diethanolamine or triethanolamine salts, and monoisopropylamine, diisopropylamine, or triisopropylamine salts; the aforementioned alkyl and acyl groups contain 6 to 24 carbon atoms, and the alkyl groups may be saturated or unsaturated; the aforementioned aryl groups are selected from phenyl or benzyl; and the aforementioned ether-containing surfactants may be surfactant molecules containing 1-10 ethylene oxide and/or propylene oxide units. Specific examples of the anionic surfactants include sodium, potassium, lithium, magnesium, or ammonium salts of lauryl alcohol polyoxyethylene ether sulfates, tridecyl alcohol polyether sulfates, myristyl alcohol polyether sulfates, C₁₂-C₁₃ palmityl alcohol polyether sulfates, C₁₂-C₁₄ palmityl alcohol polyether sulfates, or C₁₂-C₁₅ palmityl alcohol polyether sulfates, which contain 1, 2, or 3 moles of ethylene oxide ethoxylated groups, sodium, potassium, lithium, magnesium, ammonium or triethanolamine salts of lauryl sulfates, cocoyl sulfates, tridecyl sulfates, myristyl sulfates, cetyl sulfates, cetyl stearyl sulfates, stearyl sulfates, oleyl sulfates and tallow sulfates, disodium lauryl sulfosuccinate, disodium lauryl alcohol polyethoxyether sulfosuccinate, sodium cocoyl hydroxyethyl sulfonate, sodium C₁₂-C₁₄ alkene sulfonate, sodium lauryl alcohol polyethoxyether-6 carboxylate, sodium cocoyl glycerate, sodium myristyl sarcosinate, sodium dodecyl benzene sulfonate, sodium cocoyl sarcosinate, sodium cocoyl glutamate, potassium myristyl glutamate, monolauryl triethanolamine phosphate, or fatty acid soap (including sodium, potassium, ammonium, and triethanolamine salts of saturated and unsaturated fatty acids containing 8-22 carbon atoms).

Preferably, the anionic surfactants include one or more selected from sodium lauroyl sarcosinate, sodium lauroyl amino propionate, sodium cocoyl amino propionate, sodium cocoyl glutamate, and sodium C14-C16 alpha olefin sulfonate; and further preferably, the anionic surfactants are sodium lauroyl sarcosinate and/or sodium C14-C16 alpha olefin sulfonate.

The cationic conditioning agents include, but are not limited to, alkyl amines, alkyl imidazolines, ethoxylated amines, quaternary ammonium compounds, and quaternized esters or alkyl amine oxides (under the low pH condition). The alkyl amine surfactants may be salts of primary, secondary, and tertiary substituted or unsubstituted fatty C₁₂-C₂₂ alkyl amines, and materials sometimes referred to as "amidoamines".

Examples of the aforementioned alkyl amines and salts thereof include dimethyl cocoamine, dimethyl palmitamine, dioctylamine, dimethyl stearylamine, dimethyl soy amine, soy amine, myristyl amine, tridecyl amine, ethyl stearyl amine, N-tallow propane diamine, ethoxylated stearyl amine, dihydroxyethyl stearyl amine, arachidyl behenamine, dimethyl laurylamine, stearylamine hydrochloride, soy amine chlorides, stearylamine formates, N-tallow propane diamine dichlorides, or ammonia-terminated polydimethylsiloxane (whose INCI name is a silicone polymer terminated with an amino functional group, such as aminoethyl aminopropyl siloxane);
examples of the aforementioned amidoamines and salts thereof include stearoamidopropyl dimethylamine, stearoamidopropyl dimethylamine citrates, palmitoyl aminopropyl diethylamine, or cocoamidopropyl dimethylamine lactates;
examples of the aforementioned alkyl imidazoline surfactants include alkyl hydroxyethyl imidazolines, such as stearyl hydroxyethyl imidazoline, cocoyl hydroxyethyl imidazoline, and ethyl hydroxymethyl oleyl imidazoline; and
examples of the aforementioned ethoxylated amines include PEG-cocoyl polyamines, PEG-15 tallow amine, and quaternary ammonium-52.

Some of the quaternary ammonium compounds correspond to the general formula: (R₅R₆R₇R₈N⁺)E⁻, where R₅, R₆, R₇, and R₈ are independently selected from aliphatic groups with 1-22 carbon atoms, or aromatics, alkoxy, polyoxyalkylene, alkylamido, hydroxyalkyl, aryl, or alkylaryl groups with 1-22 carbon atoms in the alkyl chains; and E⁻ is a salt that forms anions, for example, it is selected from halogens (such as chloride ions and bromide ions), acetate ions, citrate ions, lactate ions, glycerate ions, phosphate ions, nitrate ions, sulfate ions, or alkyl sulfate ions. Aliphatic groups may contain ether linking groups, ester linking groups, or other groups such as amino groups in addition to carbon and hydrogen atoms. Long chain aliphatic groups, such as those with about 12 or more carbons, may be saturated or unsaturated. Aryl is selected from phenyl or benzyl.

Examples of the aforementioned quaternary ammonium compounds include cetyl trimethyl ammonium chloride, cetyl pyridinium chloride, dicacetearyl dimethyl ammonium chloride, bis (hexadecyl) dimethyl ammonium chloride, stearyl dimethyl benzyl ammonium chloride, bis (octadecyl) dimethyl ammonium chloride, bis (eicosyl) dimethyl ammonium chloride, bis (dococyl) dimethyl ammonium chloride, bis (hexadecyl) dimethyl ammonium chloride, bis (hexadecyl) dimethyl ammonium acetate, docosyl trimethyl ammonium chloride, benzalkonium chloride, benzethonium chloride, di (cocoalkyl) dimethyl ammonium chloride, ditallow dimethyl ammonium chloride, di (hydrogenated tallow) dimethyl ammonium chloride, di (hydrogenated tallow) dimethyl ammonium acetate, ditallow dimethyl ammonium methyl sulfate, ditallow dipropyl ammonium phosphate, or ditallow dimethyl ammonium nitrate.

Examples of the aforementioned amine oxides include dimethyl-dodecyl amine oxide, oleyl di (2-hydroxyethyl) amine oxide, dimethyl tetradecyl amine oxide, di (2-hydroxyethyl)-tetradecyl amine oxide, dimethyl hexadecyl amine oxide, behenamine oxide, cocoamine oxide, decyl tetradecyl amine oxide, dihydroxyethyl C12-15 alkoxy propyl amine oxide, dihydroxyethyl cocoamine oxide, dihydroxyethyl lauramine oxide, dihydroxyethyl stearamine oxide, dihydroxyethyl tallow amine oxide, hydrogenated palm kernel amine oxide, hydrogenated tallow amine oxide, hydroxyethyl hydroxypropyl C12-C15 alkoxy propyl amine oxide, lauramine oxide, myristamine oxide, cetylamine oxide, oleoamidopropyl amine oxide, oleylamine oxide, palmitamine oxide, PEG-3 lauramine oxide, dimethyl lauramine oxide, potassium triphosphoyl methylamine oxide, soy acyl aminopropylamine oxide, cocoyl aminopropylamine oxide, stearylamine oxide, or tallow amine oxide.

Preferably, the cationic conditioning agents include one or more selected from polyquaternium-10, polyquaternium-7, polyquaternium-6, polyquaternium-67, and cationic guar gum; and further preferably, polyquaternium-10 or/and cationic guar gum.

The non-ionic surfactants include, but are not limited to, aliphatic (C₆-C₁₈) primary or secondary straight-chain or branched-chain acids, alcohols, or phenols; alkyl ethoxylates; alkylphenol alkoxylates (especially ethoxylates and mixed ethoxy/propoxy moieties); block oxidized alkylene condensates of alkylphenols; oxidized alkylene condensates of alkanols; epoxy ethane/epoxy propane block copolymers, mono- or di-alkyl alkanolamides; alkyl polyglucosides (APGs); sorbitan fatty acid esters; polyoxyethylene sorbitan fatty acid esters; polyoxyethylene sorbitol esters; polyoxyethylene acid, and polyoxyethylene alcohols.

Examples of the aforementioned non-ionic surfactants include cocoyl mono- or diethanolamide, cocoyl glucoside, decyl diglucoside, lauryl diglucoside, cocoyl diglucoside, polysorbates 20, 40, 60 and 80, ethoxylated straight-chain alcohols, cetostearyl alcohols, lanolin alcohols, stearic acid, glyceryl stearate, PEG-100 stearate, lauryl alcohol polyethoxy ether 7, oleth 20, alkoxylated methyl glucosides such as methyl gluceth-10, methyl gluceth-20, PPG-10 methyl glucose ether, and PPG-20 methyl glucose ether; and hydrophobic modified alkoxylated methyl glucosides, such as PEG120 methyl glucose glycol ester, PEG-120 methyl glucose trioleate, and PEG-20 methyl glucose sesquistearate. In addition, non-ionic surfactants disclosed in US Patent Applications 6573375, 6727357, 3929678, 4565647, 5720964, and 5858948, as well as World Patent Application WO99/21530, and McCutcheon's Emulsifiers and Detergents (North American and International Editions, by Schwartz, Perry and Berch) may also serve as examples of the aforementioned non-ionic surfactants.

Preferably, the non-ionic surfactants include one or more selected from lauroyl glycerides, alkyl glycosides, polyethylene glycol stearate and cocoamide methyl monoethanolamine; and further preferably, one or more of alkyl glycosides, lauroyl glycerides, and cocoamide methyl monoethanolamine.

The zwitterionic surfactants include, but are not limited to, amino acids (e.g., N-alkyl amino acids and N-acyl amino acids), betaines, sulfobetaines, and alkyl amphoteric carboxylates.

The aforementioned amino acid-based zwitterionic surfactants include substances as shown in the following formula: 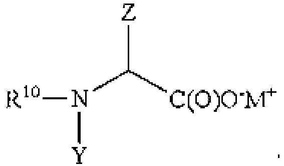 in the formula, R¹⁰ represents a saturated or unsaturated hydrocarbon group having 10-22 carbon atoms or an acyl group of a saturated or unsaturated hydrocarbon group having 9-22 carbon atoms, Y is hydrogen or methyl, and Z is selected from hydrogen, -CH₃, - CH(CH₃)₂, -CH₂CH(CH₃)₂, -CH(CH₃)CH₂CH₃, -CH₂C₆H₅, -CH₂C₆H₄OH , -CH₂OH, - CH(OH)CH₃, -(CH₂)₄NH₂, -(CH₂)₃NHC(NH)NH₂, -CH₂C(O)O⁻M⁺, and -(CH₂)₂C(O)O⁻M⁺. M is acation that forms salts. In one aspect, R¹⁰ represents a group selected from straight-chain or branched C₁₀-C₂₂ alkyl, straight-chain or branched C₁₀-C₂₂ alkenyl, and an acyl group represented by R¹¹C(O)- (where R¹¹ is selected from straight-chain or branched C₉-C₂₂ alkyl, and straight-chain or branched C₉-C₂₂ alkenyl). In one aspect, M⁺ is selected from sodium, potassium, ammonium, and triethanolamine (TEA).

Examples of the aforementioned amino acid-based zwitterionic surfactants include alanine, arginine, aspartic acid, glutamic acid, glycine, isoleucine, leucine, lysine, phenylalanine, serine, tyrosine, valine, and acylated and alkylated amino acids, such as sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, potassium myristoyl glutamate, sodium cocoyl alanine, TEA-lauroyl alanine, sodium cocoyl glycerate, potassium cocoyl glycerate, sodium lauroyl sarcosinate, sodium cocoyl sarcosinate, sodium myristyl sarcosinate, sodium oleoyl sarcosinate, ammonium lauroyl sarcosinate, and the like.

The aforementioned zwitterionic surfactants based on betaine include substances as shown in the following formula: 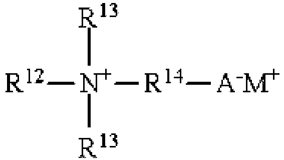 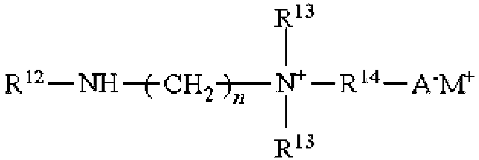 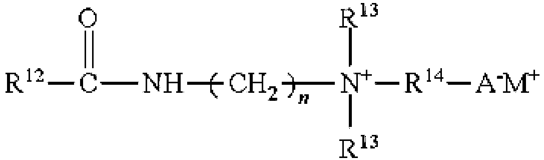 where R¹² is a C₇-C₂₂ alkyl or alkenyl group, each R¹³ is independently a C₁-C₄ alkyl group, R¹⁴ is a C₁ to C₅ alkylene group or a hydroxyl-substituted C₁ to C₅ alkylene, n is an integer from 2 to 6, A is a carboxylic acid or sulfonic acid group, and M is a salt that forms cations. In one aspect, R¹² is C₁₁-C₁₈ alkyl or C₁₁-C₁₈ alkenyl. In one aspect, R¹³ is a methyl group. In one aspect, R¹⁴ is methylene, ethylidene, or hydroxypropylene. In one aspect, n is 3. Further, M is selected from sodium, potassium, magnesium, ammonium, as well as monoethanolamine, diethanolamine, or triethanolamine cations.

Examples of the aforementioned betaine-based zwitterionic surfactants include lauryl betaine, cocoyl betaine, oleyl betaine, cocoyl hexadecyl dimethyl betaine, lauryl amidopropyl betaine, cocoyl aminopropyl betaine, or cocoyl aminopropyl hydroxysulfobetaine.

The aforementioned zwitterionic surfactants based on alkyl amphoteric carboxylates include substances as shown in the following formula: 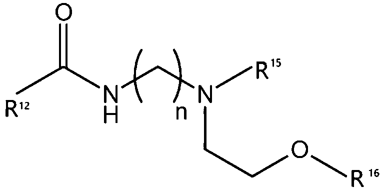 in the formula, R¹² is C₇-C₂₂ alkyl or alkenyl, R¹⁵ is -CH₂C(O)O⁻M⁺, - CH₂CH₂C(O)O⁻M⁺, or -CH₂CH(OH)CH₂SO₃⁻M⁺, R¹⁶ is hydrogen or -CH₂C(O)O⁻M⁺, and M is selected from sodium, potassium, magnesium, ammonium, as well as monoethanolamine, diethanolamine, or triethanolamine cations.

Examples of the aforementioned surfactants based on alkyl amphoteric carboxylates include sodium cocoyl amphoacetate, sodium lauryl amphoacetate, sodium octyl amphoacetate, disodium cocoyl amphoteric diacetate, disodium lauryl amphoteric diacetate, disodium octyl amphoteric diacetate, disodium octyl amphoteric diacetate, disodium cocoyl amphoteric dipropionate, disodium lauryl amphoteric dipropionate, disodium octyl amphoteric dipropionate, and disodium octyl amphoteric dipropionate.

Preferably, the zwitterionic surfactants include one or more selected from cocoamidopropyl betaine, sodium lauryl amphoacetate, cocoamido propyl hydroxy sulfobetaine, lauramide hydroxy sulfobetaine, and lauramide propyl betaine; and further preferably, one or more of cocoamidopropyl betaine, sodium lauryl amphoacetate, and cocoamido propyl hydroxy sulfobetaine.

Specifically, the weight percentage of the surfactant C in the cleaning composition is 0-15%.

Preferably, the cleaning composition further includes at least one of high-molecular polymers, hydrophobic skin or hair benefiting agents, water-soluble skin or hair benefiting agents, exfoliants, and additives, where the additives are selected from at least one of preservatives, chelating agents, antimicrobial agents, fillers (such as clay, and talc), penetrating agents, opacifying agents, fragrances, dispersing agents, and film forming agents.

The high-molecular polymers include, but are not limited to, water-soluble and/or water dispersible polymers, and cationic, anionic, amphoteric or non-ionic polymers. Examples of the aforementioned high-molecular polymers include high molecular weight polyethylene glycol, such as Polyox^{®} WSR-205 (PEG 14M), Polyox^{®} WSR-N-60K (PEG 45M), or Polyox^{®} WSR-301 (PEG 90M); carbohydrate gum, such as cellulose gum, microcrystalline cellulose, cellulose gel, hydroxyethyl cellulose, hydroxypropyl cellulose, sodium carboxymethyl cellulose, hydroxymethyl or carboxymethyl cellulose, methyl cellulose, ethyl cellulose, guar gum, karaya gum, tragacanth gum, Arabic gum, gumacavia, agaropectin, and/or xanthan gum; modified or unmodified starch granules with a gelatinization temperature of 30°C to 85°C, or pregelatinized cold water soluble starch; polyacrylates; Carbopols; alkaline soluble emulsion polymers, such as Aculyn 28, Aculyn 22 or Carbopol Aqua SF1; cationic polymers, such as modified polysaccharides, including cationic guar gums available from Rhone Poulenc under the trade names Jaguar C13S, Jaguar C14S, Jaguar C17 or Jaguar C16, from Lamberti under the trade name BF Guar C17, or from Aqualon under the trade names Aqua D4091 or Aqua D4051; cationically modified cellulose such as UCARE Polymer JR30 or JR 40 from Amerchol, and N-Hance 3000, N-Hance 3196, N-Hance CPX215 or N-Hance GPX 196 from Hercules; synthetic cationic polymers, such as Nalco's Merquat 100, Merquat 280, Merquat 281 or Merquat 550; cationic starch, such as StaLok^{®} 100, 200, 300, or 400 prepared by Staley Inc.; cationic galactomannan based on Galactasol 800 series guar gum from Henkel, Inc.; and Quadrisect Um-200 or Polyquaternium-24.

Specifically, the weight percentage of the high-molecular polymer in the cleaning composition is 0-10%.

Examples of the hydrophobic skin or hair benefiting agents include, but are not limited to, the following ingredients:
(a) silicone oil and its modifiers, such as linear and cyclic polydimethylsiloxane; amino, alkyl, alkyl aryl, or aryl silicone oil;
(b) fats and oils, including natural fats and oils (triglycerides), such as jojoba oil, soybean oil, sunflower oil, rice bran oil, avocado oil, almond oil, olive oil, sesame oil, apricot kernel oil, castor oil, coconut oil, mink oil, cocoa butter, tallow oil, and lard; hardened oils obtained by performing hydrogenation on the above oils; synthetic monoglycerides, diglycerides and triglycerides, such as glyceryl myristate and glyceryl 2-ethylhexanoate;
(c) waxes, such as carnauba wax, spermaceti wax, beeswax, lanolin, or their derivatives;
(d) hydrophobic plant extracts;
(e) hydrocarbons, such as petrolatum, polybutene, liquid paraffin, microcrystalline wax, ceresin, squalene, pristan, or mineral oil;
(f) higher alcohols, such as lauryl alcohol, cetanol, stearyl alcohol, oleyl alcohol, behenyl alcohol, cholesterol, or 2-hexyl-1-decanol;
(g) esters, such as cetearyl octanoate, myristyl lactate, cetearyl lactate, isopropyl myristate, myristyl myristate, isopropyl palmitate, isopropyl adipate, butyl stearate, decyl oleate, cholesterol isostearate, glyceryl monostearate, glyceryl distearate, glyceryl tristearate, alkyl lactate, alkyl citrate, or alkyl tartrate;
(h) essential oils and their extracts, such as mentha oil, jasmine oil, camphor oil, white fir oil, bitter orange peel oil, ryu, turpentine oil, cinnamon oil, bergamot oil, tangerine oil, calamus oil, pine oil, lavender oil, bay, clove oil, hiba, eucalyptus oil, lemon oil, borage oil, thyme oil, peppermint oil, rose oil, sage oil, sesame oil, ginger oil, basil oil, juniper oil, lemongrass oil, rosemary oil, rosewood oil, avocado oil, grape oil, grape seed oil, myrrh oil, cucumber oil, watercress oil, calendula oil, elderberry oil, geranium oil, linden flower oil, amaranth oil, seaweed oil, ginkgo oil, ginseng oil, carrot oil, guarana oil, tea tree oil, jojoba oil, comfrey oil, oat oil, cocoa oil, neroli oil, vanilla oil, green tea oil, pennyroyal, aloe vera oil, menthol oil, eucalyptol oil, eugenol oil, citral oil, citronelle oil, borneol oil, linalool oil, geraniol oil, evening primrose oil, camphor oil, thymol oil, spirantol, penene, limonene oil, or terpenoid oils; and
(i) mixtures of any of the above components, and the like.

Specifically, the hydrophobic skin or hair benefiting agents account for 0-30% by weight of the cleaning composition; preferably, the hydrophobic skin or hair benefiting agents account for 0.1-10% by weight of the cleaning composition; and more preferably, the hydrophobic skin or hair benefiting agents account for 0.1-5% by weight of the cleaning composition.

Examples of the water-soluble skin or hair benefiting agents include, but are not limited to, the following ingredients:
allantoin, urea, pyrrolidone carboxylic acid and its salts, hyaluronic acid and its salts, sorbic acid and its salts, amino acids (such as lysine, arginine, cystine, guanidine), C₃-C₆ polyols such as glycerol, propylene glycol, hexanediol, hexanetriol, ethoxy diglycol, saccharides and saccharide alcohols, including glucose, sucrose, fructose, galactose, xylose, mannose, arabinose, ribulose, ribose and other monosaccharides with carbon numbers of 5-6, sorbitol, inositol, mannitol, maltitol and other saccharide alcohols, starch and starch derivatives, panthenol such as dl-panthenol, lactamide monoethanolamine or acetamide monoethanolamine, or their mixtures.

Specifically, the water-soluble skin or hair benefiting agents account for 0-50% by weight of the cleaning composition; and preferably, the water-soluble skin or hair benefiting agents account for 1-30% by weight of the cleaning composition.

The exfoliants are hard particles which are undefined in shapes and have an average diameter greater than 50 microns and less than a few millimeters, and their functions are to help remove dry skin. Examples of the aforementioned exfoliants include, but are not limited to, silica, talc, calcite, pumice, or tricalcium phosphate; seeds such as rice seeds, apricot seeds, and the like; crushed shells such as almond shells and walnut shells; oats; polymers such as polyethylene and polypropylene beads, petals, or leaves; microcrystalline wax beads; jojoba ester beads, and the like.

Specifically, the exfoliants account for 0-45% by weight of the cleaning composition; and preferably, the exfoliants account for 1-10% by weight of the cleaning composition.

A preparation method of the above-mentioned cleaning composition is a conventional method in the art, and the preparation method includes:
(1) adding alkanoyl taurine salt, methyl alkanoyl taurine salt, other surfactants and solvents into a container, mixing well, and then heating and stirring for dissolving so as to obtain a component A; and
(2) after the component A is cooled down, adding the remaining components, and stirring and mixing evenly to obtain a cleaning composition.

In yet another aspect, the present application also provides use of the above cleaning composition in the preparation of personal care cleaners.

The personal care cleaners include one or more of shampoo, shower gel, scrub cream and facial cleanser.

The personal care cleaners also include one or more of emulsifiers, conditioning agents, emollients, moisturizers, humectants, lubricants, chelating agents, fillers, antioxidants, preservatives, active ingredients, fragrances, dyes, buffering agents, exfoliating agents, pH regulators, inorganic salts, solvents, viscosity regulators, and opacifying agents.

The emollients, moisturizers, humectants, lubricants, chelating agents, fillers, antioxidants, preservatives, active ingredients, fragrances, dyes, buffering agents, exfoliating agents, pH regulators, inorganic salts, solvents, viscosity regulators, and opacifying agents mentioned above are all conventional components in the art, and are not limited herein.

Compared with the prior art, the present application has the following beneficial effects.
(1) According to the present application, the cleaning composition is prepared by mixing the alkanoyl taurine salt with the methyl alkanoyl taurine salt. By controlling the ratio of the alkanoyl taurine salt and the methyl alkanoyl taurine salt, the paste-forming points thereof are reduced, which can solve the problems that the alkanoyl taurine salt and the methyl alkanoyl taurine salt have an unstable state of forming paste in winter, dissolving in summer, and being half-dissolved and half-pasted in spring and autumn.
(2) According to the present application, the cleaning composition is prepared by mixing the alkanoyl taurine salt with the methyl alkanoyl taurine salt. By controlling the ratio of the alkanoyl taurine salt and the methyl alkanoyl taurine salt, the effects of viscosity, transparency at room temperature, and transparency at low temperature can be achieved at the same time, thus meeting the basic needs of the current market for silicone-free and sulfate-free shampoos.
(3) According to the present application, the cleaning composition is prepared by mixing the alkanoyl taurine salt with the methyl alkanoyl taurine salt, which can combine the advantages of the alkanoyl taurine salt and the methyl alkanoyl taurine salt at the same time, so that the viscosity is appropriate, the paste is soft and easy to spread, and the composition is good in high and low temperature stability.

### DETAILED DESCRIPTION OF THE EMBODIMENTS

The present disclosure will be further illustrated with reference to the following examples, but these examples do not constitute any limitation to the present disclosure.
1. Purchase methods and models of the reagents used in the examples

| Raw material | Model | Manufacturer |
|---|---|---|
| Sodium lauroyl taurate | 95% | Zhangjiagang Great Chemical Co., Ltd. |
| Sodium methyl lauroyl taurate | 95% | |
| Sodium methyl myristoyl taurate | 95% | |
| Sodium methyl cocoyl taurate | 95% | |
| Sodium myristoyl taurate | 95% | |
| Sodium cocoyl taurate | 95% | |
| Cocoamido propyl hydroxy sulfobetaine | 30% | Guangzhou Tinci |
| Polyquaternium-7 | M550 | |
| PEG-150 stearate | 638 | Daoming Chemical |
| Citric acid | Citric acid monohydrate | Ensign |
| C14-16 sodium alpha olefin sulfonate | 35% | Sinolight Surfactants Technology Co., Ltd. |
| Sodium chloride | Iodine-free table salt | Zhangjiagang Salt Industry Company |
| Glycerol | 99.5% | Zhangjiagang Taiko |
| Sorbitol | 100% | Roquette |

2. Detection methods used in examples of the present disclosure

| Test item | Detection method |
|---|---|
| Appearance, 25°C | A sample was placed at 25°C and maintained at the consistent temperature for 48 hours, and whether the appearance was pasted or not was visually inspected. |
| Appearance, 10°C | A sample was placed at 10°C and maintained at the consistent temperature for 48 hours, and whether the appearance was pasted or not was visually inspected. |
| Dissolution temperature, °C | A sample was placed at -18°C to form a paste, and then kept at 0°C for 24 hours; after that, the sample was heated up at a rate of 5°C/hour for observation of dissolution temperature; and the complete dissolution temperature was recorded. |
| Paste spreadability at 25°C | After a sample was placed at 25°C and maintained at the consistent temperature for 48 hours, 1g of the sample was extruded onto wet hands and rubbed for 10 times with the hands to observe whether it was completely spread; if there was particle residue, or blocky or soapy residue, it was proved to be unqualified; and if it was completely spread or completely dissolved, it was proved to be qualified. |
| Viscosity at 25°C | A sample was placed at 25°C and maintained at the consistent temperature for 2 hours, and then the viscosity was measured using a rotary viscometer. |
| Transparent or not at 25°C | A sample was placed at 25°C and maintained at the consistent temperature for 24 hours before visual inspection; the light transmittance was measured using a spectrophotometer; if the light transmittance was greater than 90% at a wavelength of 600nm in a 10mm glass dish, it was considered transparent; and otherwise, it was considered non-transparent. |
| Transparent or not at 5°C | A sample was placed at 5°C and maintained at the consistent temperature for 24 hours before visual inspection; the light transmittance was measured using a spectrophotometer; if the light transmittance was greater than 90% at a wavelength of 600nm in a 10mm glass dish, it was considered transparent; and otherwise, it was considered non-transparent. |
| High temperature investigation | A sample was placed in a 48°C incubator and investigated for 3 months. During this period, observation was carried out once a week. If the sample was stratified, watered, roughened, discolored and smelly, it was recorded as unqualified; and otherwise, it was recorded as qualified. |
| Low temperature investigation | A sample was placed in a 0°C incubator and investigated for 3 months. During this period, observation was carried out once a week. If the sample was stratified, watered, roughened, unable to spread, discolored and smelly, it was recorded as unqualified; and otherwise, it was recorded as qualified. |

### Examples A1-A9 Cleaning composition containing surfactant system and its preparation method

The component formula is:

| | Sodium lauroyl taurate | Sodium methyl lauroyl taurate | Water |
|---|---|---|---|
| Raw material specification | 95% | 95% | 100% |
| Example A1 | 27 | 3 | 70 |
| Example A2 | 24 | 6 | 70 |
| Example A3 | 21 | 9 | 70 |
| Example A4 | 18 | 12 | 70 |
| Example A5 | 15 | 15 | 70 |
| Example A6 | 12 | 18 | 70 |
| Example A7 | 9 | 21 | 70 |
| Example A8 | 6 | 24 | 70 |
| Example A9 | 3 | 27 | 70 |

### Preparation method:

Sodium lauroyl taurate, sodium methyl lauroyl taurate and water in the formulated amounts were added, heated up to 50°C and dissolved by stirring, so that a cleaning composition was obtained.

### Comparative Examples A1-A2 Cleaning composition containing surfactant system and its preparation method

The component formula is:

| | Raw material specification | Comparative Example A1 | Comparative Example A2 |
|---|---|---|---|
| Sodium lauroyl taurate | 95% | 30 | 0 |
| Sodium methyl lauroyl taurate | 95% | 0 | 30 |
| Water | 100% | 70 | 70 |

### Preparation method:

Sodium lauroyl taurate, sodium methyl lauroyl taurate and water in the formulated amounts were added, heated up to 50°C and dissolved by stirring, so that a cleaning composition was obtained.

Performance test: The test results are shown in Tables 1-2 below.

**Table 1 Relevant performance test results of the cleaning compositions prepared in Examples A1-A9**

| | Appearance, 25°C | Appearance, 10°C | Dissolution temperature, °C | Paste spreadability at 25°C |
|---|---|---|---|---|
| Example A1 | White paste | White paste | 33.3 | × |
| Example A2 | Transparent liquid | White paste | 22 | ○ |
| Example A3 | Transparent liquid | White paste | 15.4 | ○ |
| Example A4 | Transparent liquid | Transparent liquid | 9.5 | ○ |
| Example A5 | Transparent liquid | Transparent liquid | 5.3 | ○ |
| Example A6 | Transparent liquid | Transparent liquid | 7.7 | ○ |
| Example A7 | Transparent liquid | White paste | 12.2 | ○ |
| Example A8 | Transparent liquid | White paste | 17.6 | ○ |
| Example A9 | Transparent liquid | White paste | 19.1 | ○ |

**Table 2 Relevant performance test results of the cleaning compositions prepared in Comparative Examples A1-A2**

| | Comparative Example A1 | Comparative Example A2 |
|---|---|---|
| Appearance, 25°C | White paste | Transparent liquid |
| Appearance, 10°C | White paste | White paste |
| Dissolution temperature, °C | 35.5 | 20.4 |
| Paste spreadability at 25°C | × | ○ |

As can be seen from Tables 1-2 above, the dissolution temperature of Comparative Example A1 reaches 35°C, while the dissolution temperature of Comparative Example A2 is above 20°C. When the ratio of sodium lauroyl taurate to sodium methyl lauroyl taurate is within a range from 7:3 to 3:7, the dissolution temperature is reduced to below 15°C, indicating that the prepared cleaning composition can be maintained in a liquid state throughout the year at room temperature and thus is convenient for use. Furthermore, it can be seen that due to the combination of alkanoyl taurine salt and methyl alkanoyl taurine salt, the paste-forming point is significantly reduced, and the convenience of use is improved.

### Examples B1-B9 Cleaning composition containing surfactant system and its preparation method

The component formula is:

| | Sodium lauroyl taurate | Sodium methyl myristoyl taurate | Water |
|---|---|---|---|
| Raw material specification | 95% | 95% | 100% |
| Example B1 | 27 | 3 | 70 |
| Example B2 | 24 | 6 | 70 |
| Example B3 | 21 | 9 | 70 |
| Example B4 | 18 | 12 | 70 |
| Example B5 | 15 | 15 | 70 |
| Example B6 | 12 | 18 | 70 |
| Example B7 | 9 | 21 | 70 |
| Example B8 | 6 | 24 | 70 |
| Example B9 | 3 | 27 | 70 |

### Preparation method:

Sodium lauroyl taurate, sodium methyl myristoyl taurate and water in the formulated amounts were added, heated up to 50°C and dissolved by stirring, so that a cleaning composition was obtained.

### Comparative Examples B1-B2 Cleaning composition containing surfactant system and its preparation method

The component formula is:

| | Raw material specification | Comparative Example B1 | Comparative Example B2 |
|---|---|---|---|
| Sodium lauroyl taurate | 95% | 30 | 0 |
| Sodium methyl myristoyl taurate | 95% | 0 | 30 |
| Water | 100% | 70 | 70 |

### Preparation method:

Sodium lauroyl taurate, sodium methyl myristoyl taurate and water in the formulated amounts were added, heated up to 50°C and dissolved by stirring, so that a cleaning composition was obtained.

Performance test: The test results are shown in Tables 3-4 below.

**Table 3 Relevant performance test results of the cleaning compositions prepared in Examples B1-B9**

| | Appearance, 25°C | Appearance, 10°C | Dissolution temperature, °C | Paste spreadability at 25°C |
|---|---|---|---|---|
| Example B1 | White paste | White paste | 34.7 | × |
| Example B2 | White paste | White paste | 25.7 | ○ |
| Example B3 | Transparent liquid | White paste | 16 | ○ |
| Example B4 | Transparent liquid | White paste | 12.2 | ○ |
| Example B5 | Transparent liquid | White paste | 13.1 | ○ |
| Example B6 | Transparent liquid | White paste | 15.7 | ○ |
| Example B7 | Transparent liquid | White paste | 18.3 | ○ |
| Example B8 | Transparent liquid | White paste | 24.2 | ○ |
| Example B9 | White paste | White paste | 31.1 | ○ |

**Table 4 Relevant performance test results of the cleaning compositions prepared in Comparative Examples B1-B2**

| | Comparative Example B1 | Comparative Example B2 |
|---|---|---|
| Appearance, 25°C | White paste | White paste |
| Appearance, 10°C | White paste | White paste |
| Dissolution temperature, °C | 35.5 | 37.2 |
| Paste spreadability at 25°C | × | × |

As can be seen from Tables 3-4 above, the dissolution temperatures of Comparative Examples B1 and B2 reach above 35°C. When the ratio of sodium lauroyl taurate to sodium methyl myristoyl taurate is within a range from 7:3 to 3:7, the dissolution temperature is reduced to below 20°C, indicating that the prepared cleaning composition can be maintained in a liquid state throughout the year at room temperature and thus is convenient for use. Furthermore, it can be seen that due to the combination of alkanoyl taurine salt and methyl alkanoyl taurine salt, the paste-forming point is significantly reduced, and the convenience of use is improved.

### Examples C1-C9 Cleaning composition containing surfactant system and its preparation method

The component formula is:

| | Sodium lauroyl taurate | Sodium methyl cocoyl taurate | Water |
|---|---|---|---|
| Raw material specification | 95% | 95% | 100% |
| Example C1 | 27 | 3 | 70 |
| Example C2 | 24 | 6 | 70 |
| Example C3 | 21 | 9 | 70 |
| Example C4 | 18 | 12 | 70 |
| Example C5 | 15 | 15 | 70 |
| Example C6 | 12 | 18 | 70 |
| Example C7 | 9 | 21 | 70 |
| Example C8 | 6 | 24 | 70 |
| Example C9 | 3 | 27 | 70 |

### Comparative Examples C1-C2 Cleaning composition containing surfactant system and its preparation method

The component formula is:

| | Raw material specification | Comparative Example C1 | Comparative Example C2 |
|---|---|---|---|
| Sodium lauroyl taurate | 95% | 30 | 0 |
| Sodium methyl cocoyl taurate | 95% | 0 | 30 |
| Water | 100% | 70 | 70 |

### Preparation method:

Sodium lauroyl taurate, sodium methyl cocoyl taurate and water in the formulated amounts were added, heated up to 50°C and dissolved by stirring, so that a cleaning composition was obtained.

Performance test: The test results are shown in Tables 5-6 below.

**Table 5 Relevant performance test results of the cleaning compositions prepared in Examples C1-C9**

| | Appearance, 25°C | Appearance, 10°C | Dissolution temperature, °C | Paste spreadability at 25°C |
|---|---|---|---|---|
| Example C1 | White paste | White paste | 34.9 | × |
| Example C2 | Transparent liquid | White paste | 23 | ○ |
| Example C3 | Transparent liquid | White paste | 15.9 | ○ |
| Example C4 | Transparent liquid | Transparent liquid | 9.7 | ○ |
| Example C5 | Transparent liquid | Transparent liquid | 9 | ○ |
| Example C6 | Transparent liquid | White paste | 11.2 | ○ |
| Example C7 | Transparent liquid | White paste | 15.1 | ○ |
| Example C8 | Transparent liquid | White paste | 20.5 | ○ |
| Example C9 | White paste | White paste | 25.8 | ○ |

**Table 6 Relevant performance test results of the cleaning compositions prepared in Comparative Examples C1-C2**

| | Comparative Example C1 | Comparative Example C2 |
|---|---|---|
| Appearance, 25°C | White paste | White paste |
| Appearance, 10°C | White paste | White paste |
| Dissolution temperature, °C | 35.5 | 31.5 |
| Paste spreadability at 25°C | × | × |

As can be seen from Tables 5-6 above, the dissolution temperatures of Comparative Examples C1 and C2 reach above 30°C. When the ratio of sodium lauroyl taurate to sodium methyl cocoyl taurate is within a range from 7:3 to 3:7, the dissolution temperature is reduced to below 20°C, indicating that the prepared cleaning composition can be maintained in a liquid state throughout the year at room temperature and thus is convenient for use. Furthermore, it can be seen that due to the combination of alkanoyl taurine salt and methyl alkanoyl taurine salt, the paste-forming point is significantly reduced, and the convenience of use is improved.

### Examples D1-D9 Cleaning composition containing surfactant system and its preparation method

The component formula is:

| | Sodium myristoyl taurate | Sodium methyl lauroyl taurate | Water |
|---|---|---|---|
| Raw material specification | 95% | 95% | 100% |
| Example D1 | 27 | 3 | 70 |
| Example D2 | 24 | 6 | 70 |
| Example D3 | 21 | 9 | 70 |
| Example D4 | 18 | 12 | 70 |
| Example D5 | 15 | 15 | 70 |
| Example D6 | 12 | 18 | 70 |
| Example D7 | 9 | 21 | 70 |
| Example D8 | 6 | 24 | 70 |
| Example D9 | 3 | 27 | 70 |

### Comparative Examples D1-D2 Cleaning composition containing surfactant system and its preparation method

The component formula is:

| | Raw material specification | Comparative Example D 1 | Comparative Example D2 |
|---|---|---|---|
| Sodium myristoyl taurate | 95% | 30 | 0 |
| Sodium methyl lauroyl taurate | 95% | 0 | 30 |
| Water | 100% | 70 | 70 |

### Preparation method:

Sodium myristoyl taurate, sodium methyl lauroyl taurate and water in the formulated amounts were added, heated up to 50°C and dissolved by stirring, so that a cleaning composition was obtained.

Performance test: The test results are shown in Tables 7-8 below.

**Table 7 Relevant performance test results of the cleaning compositions prepared in Examples D1-D9**

| | Appearance, 25°C | Appearance, 10°C | Dissolution temperature, °C | Paste spreadability at 25°C |
|---|---|---|---|---|
| Example D1 | White paste | White paste | 41.4 | × |
| Example D2 | White paste | White paste | 31.1 | ○ |
| Example D3 | Transparent liquid | White paste | 24.4 | ○ |
| Example D4 | Transparent liquid | White paste | 19.9 | ○ |
| Example D5 | Transparent liquid | White paste | 16.8 | ○ |
| Example D6 | Transparent liquid | White paste | 15 | ○ |
| Example D7 | Transparent liquid | White paste | 14.1 | ○ |
| Example D8 | Transparent liquid | White paste | 18.8 | ○ |
| Example D9 | Transparent liquid | White paste | 19.9 | ○ |

**Table 8 Relevant performance test results of the cleaning compositions prepared in Comparative Examples D1-D2**

| | Comparative Example D1 | Comparative Example D2 |
|---|---|---|
| Appearance, 25°C | White paste | Transparent liquid |
| Appearance, 10°C | White paste | White paste |
| Dissolution temperature, °C | 42.4 | 20.4 |
| Paste spreadability at 25°C | × | ○ |

As can be seen from Tables 7-8, the dissolution temperature of Comparative Example D1 reaches above 45°C, while the dissolution temperature of Comparative Example D2 is above 20°C. When the ratio of sodium myristoyl taurate to sodium methyl lauroyl taurate is within a range from 6:4 to 1:9, the dissolution temperature is reduced to below 20°C, indicating that the prepared cleaning composition can be maintained in a liquid state throughout the year at room temperature and thus is convenient for use. Furthermore, it can be seen that due to the combination of alkanoyl taurine salt and methyl alkanoyl taurine salt, the paste-forming point is significantly reduced, and the convenience of use is improved.

### Examples E1-E9 Cleaning composition containing surfactant system and its preparation method

The component formula is:

| | Sodium cocoyl taurate | Sodium methyl lauroyl taurate | Water |
|---|---|---|---|
| Raw material specification | 95% | 95% | 100% |
| Example E1 | 27 | 3 | 70 |
| Example E2 | 24 | 6 | 70 |
| Example E3 | 21 | 9 | 70 |
| Example E4 | 18 | 12 | 70 |
| Example E5 | 15 | 15 | 70 |
| Example E6 | 12 | 18 | 70 |
| Example E7 | 9 | 21 | 70 |
| Example E8 | 6 | 24 | 70 |
| Example E9 | 3 | 27 | 70 |

### Comparative Examples E1-E2 Cleaning composition containing surfactant system and its preparation method

The component formula is:

| | Raw material specification | Comparative Example E1 | Comparative Example E2 |
|---|---|---|---|
| Sodium cocoyl taurate | 95% | 30 | 0 |
| Sodium methyl lauroyl taurate | 95% | 0 | 30 |
| Water | 100% | 70 | 70 |

### Preparation method:

Sodium cocoyl taurate, sodium methyl lauroyl taurate and water in the formulated amounts were added, heated up to 50°C and dissolved by stirring, so that a cleaning composition was obtained.

Performance test: The test results are shown in Tables 9-10 below.

**Table 9 Relevant performance test results of the cleaning compositions prepared in Examples E1-E9**

| | Appearance, 25°C | Appearance, 10°C | Dissolution temperature, °C | Paste spreadability at 25°C |
|---|---|---|---|---|
| Example E1 | White paste | White paste | 36.3 | × |
| Example E2 | White paste | White paste | 29.5 | ○ |
| Example E3 | Transparent liquid | White paste | 22.5 | ○ |
| Example E4 | Transparent liquid | White paste | 17 | ○ |
| Example E5 | Transparent liquid | White paste | 12.6 | ○ |
| Example E6 | Transparent liquid | White paste | 10.3 | ○ |
| Example E7 | Transparent liquid | White paste | 13.2 | ○ |
| Example E8 | Transparent liquid | White paste | 17.3 | ○ |
| Example E9 | Transparent liquid | White paste | 19.6 | ○ |

**Table 10 Relevant performance test results of the cleaning compositions prepared in Comparative Examples E1-E2**

| | Comparative Example E1 | Comparative Example E2 |
|---|---|---|
| Appearance, 25°C | White paste | Transparent liquid |
| Appearance, 10°C | White paste | White paste |
| Dissolution temperature, °C | 38.1 | 20.4 |
| Paste spreadability at 25°C | × | ○ |

As can be seen from Tables 9-10, the dissolution temperature of Comparative Example E1 reaches above 35°C, while the dissolution temperature of Comparative Example E2 is above 20°C. When the ratio of sodium lauroyl taurate to sodium methyl myristoyl taurate is within a range from 6:4 to 1:9, the dissolution temperature is reduced to below 20°C, indicating that the prepared cleaning composition can be maintained in a liquid state throughout the year at room temperature and thus is convenient for use. Furthermore, it can be seen that due to the combination of alkanoyl taurine salt and methyl alkanoyl taurine salt, the paste-forming point is significantly reduced, and the convenience of use is improved.

### Application Examples A1-A9 Sulfate-free and silicon-free transparent shampoo and preparation method thereof

The component formula is:

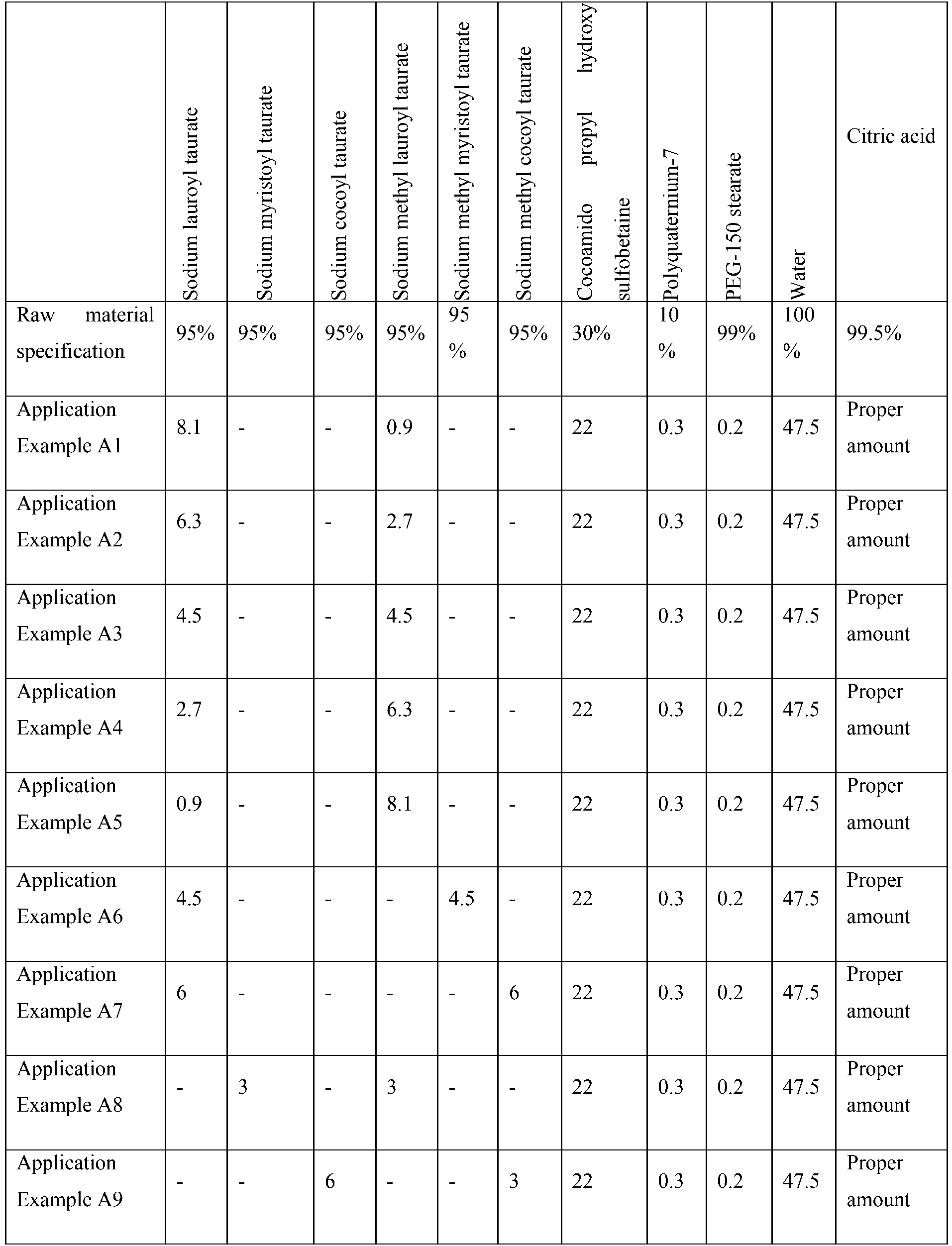

Preparation method:
(1) Sodium lauroyl taurate, sodium myristoyl taurate, sodium cocoyl taurate, sodium methyl lauroyl taurate, sodium methyl myristoyl taurate, sodium methyl cocoyl taurate, cocoamido propyl hydroxy sulfobetaine, water and PEG-150 stearate were added, and heated and stirred for dissolving, so that a component A was obtained.
(2) After cooling, the remaining components were added into the component A, and the mixture was stirred and mixed evenly, so that the shampoo was obtained.

### Application Comparative Examples A1-A6 Sulfate-free and silicon-free transparent shampoo and preparation method thereof

The component formula is:

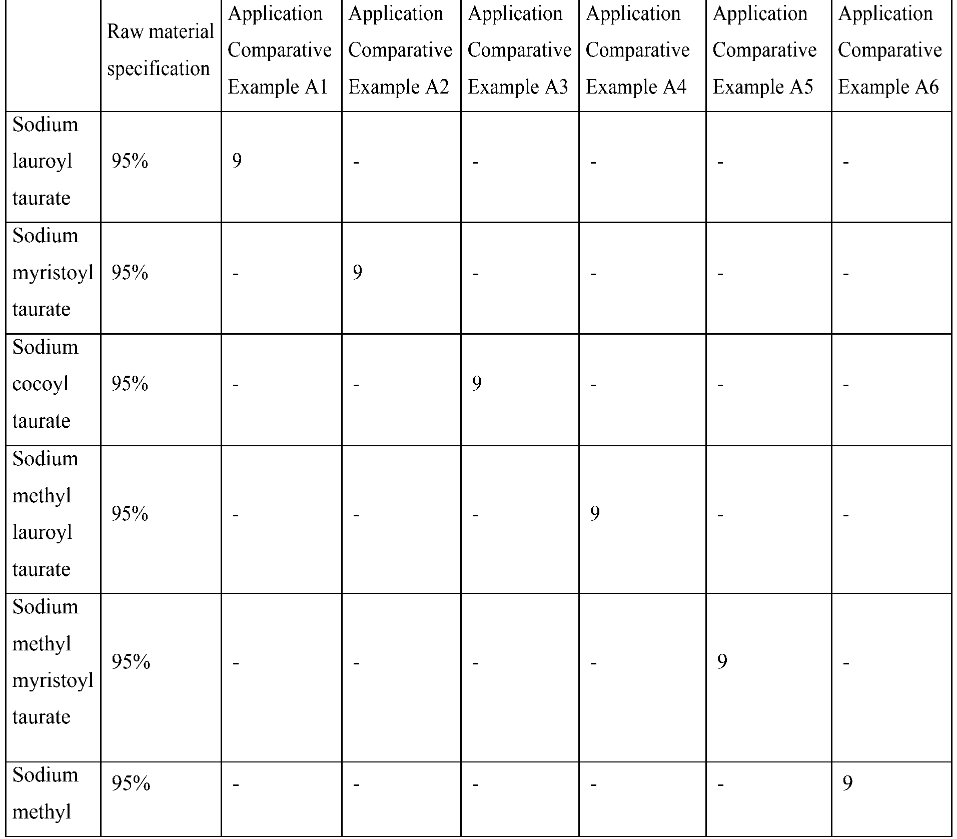

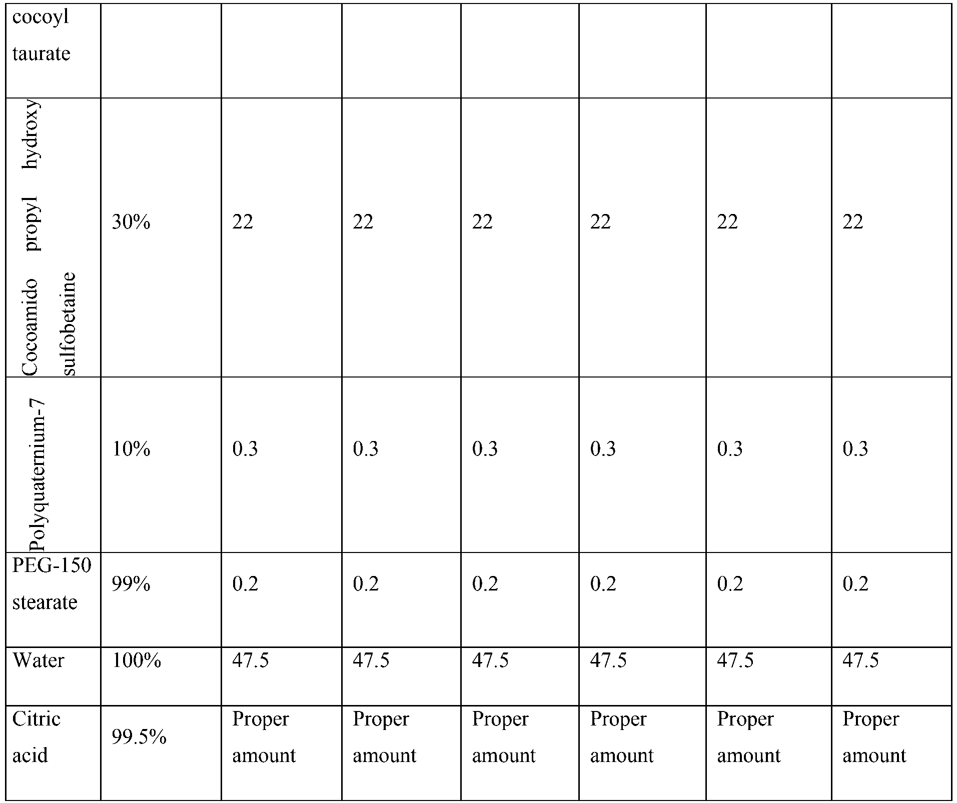

Preparation method:
(1) Sodium lauroyl taurate, sodium myristoyl taurate, sodium cocoyl taurate, sodium methyl lauroyl taurate, sodium methyl myristoyl taurate, sodium methyl cocoyl taurate, cocoamido propyl hydroxy sulfobetaine, water and PEG-150 stearate were added, and heated and stirred for dissolving, so that a component A was obtained.
(2) After cooling, the remaining components were added into the component A, and the mixture was stirred and mixed evenly, so that the shampoo was obtained.

Performance test: The test results are shown in Tables 11-12 below.

**Table 11 Relevant performance test results of the sulfate-free and silicon-free transparent shampoo prepared in Application Examples A1-A9**

| | Viscosity at 25°C | Transparent or not at 25°C | Transparent or not at 5°C |
|---|---|---|---|
| Application Example A1 | 17600 | ○ | ○ |
| Application Example A2 | 10500 | ○ | ○ |
| Application Example A3 | 8100 | ○ | ○ |
| Application Example A4 | 6300 | ○ | ○ |
| Application Example A5 | 2050 | ○ | ○ |
| Application Example A6 | 12200 | ○ | ○ |
| Application Example A7 | 11000 | ○ | ○ |
| Application Example A8 | 21500 | ○ | ○ |
| Application Example A9 | 10600 | ○ | ○ |

**Table 12 Relevant performance test results of the sulfate-free and silicon-free transparent shampoo prepared in Application Comparative Examples A1-A6**

| | Viscosity at 25°C | Transparent or not at 25°C | Transparent or not at 5°C |
|---|---|---|---|
| Application Comparative Example A1 | 25000 | ○ | × |
| Application Comparative Example A2 | 63600 | × | × |
| Application Comparative Example A3 | 34500 | ○ | × |
| Application Comparative Example A4 | 425 | ○ | × |
| Application Comparative Example A5 | 750 | × | × |
| Application Comparative Example A6 | 640 | ○ | × |

As can be seen from the descriptions in the above Tables 11-12, using either alkanoyl taurine salt or methyl alkanoyl taurine salt alone always makes it difficult to achieve both viscosity and transparency. By using alkanoyl taurine salt in combination with methyl alkanoyl taurine salt, the effects of viscosity, transparency at normal temperature and transparency at low temperature can be achieved at the same time, so that the basic needs of the current market for silicon-free and sulfate-free shampoo are met.

### Application Examples B1-B8 Sea salt scrub cream and preparation method thereof

The component formula is:

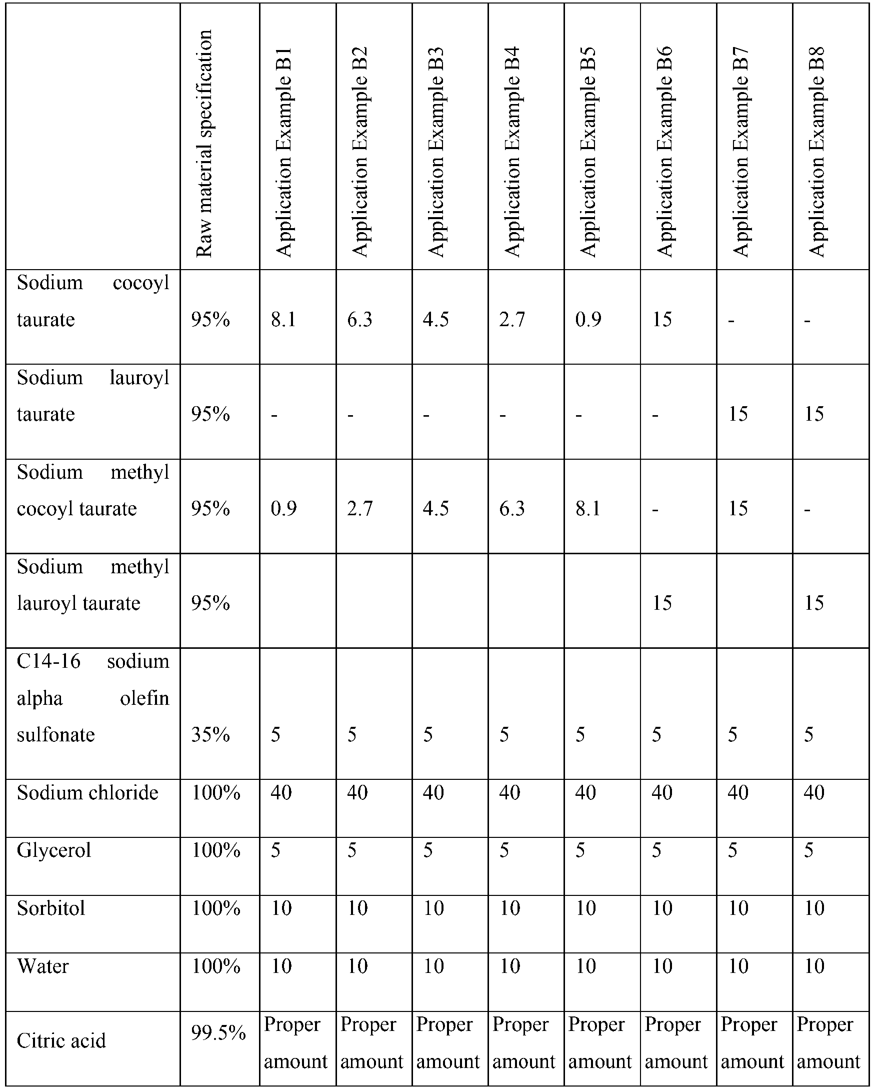

Preparation method:
(1) Sodium cocoyl taurate, sodium lauroyl taurate, sodium methyl cocoyl taurate, sodium methyl lauroyl taurate, C14-16 sodium alpha olefin sulfonate, water, glycerol, sorbitol, and sodium chloride were added, and heated and stirred for dissolving, so that a component A was obtained.
(2) After cooling, the remaining components were added into the component A, and the mixture was stirred and mixed evenly, so that the sea salt scrub cream was obtained.

**Application Comparative Examples B1-B4** Sea salt scrub cream and preparation method thereof

| | Raw material specification | Application Comparative Example B1 | Application Comparative Example B2 | Application Comparative Example B3 | Application Comparative Example B4 |
|---|---|---|---|---|---|
| Sodium cocoyl taurate | 95% | 9 | - | - | - |
| Sodium lauroyl taurate | 95% | - | 9 | - | - |
| Sodium methyl cocoyl taurate | 95% | - | - | 9 | - |
| Sodium methyl lauroyl taurate | 95% | | - | - | 9 |
| C14-16 sodium alpha olefin sulfonate | 35% | 5 | 5 | 5 | 5 |
| Sodium chloride | 100% | 40 | 40 | 40 | 40 |
| Glycerol | 100% | 5 | 5 | 5 | 5 |
| Sorbitol | 100% | 10 | 10 | 10 | 10 |
| Water | 100% | 10 | 10 | 10 | 10 |
| Citric acid | 99.5% | Proper amount | Proper amount | Proper amount | Proper amount |

Preparation method:
(1) Sodium cocoyl taurate, sodium lauroyl taurate, sodium methyl cocoyl taurate or sodium methyl lauroyl taurate, C14-16 sodium alpha olefin sulfonate, water, glycerol, sorbitol, and sodium chloride were respectively added, and heated and stirred for dissolving, so that a component A was obtained.
(2) After cooling, the remaining components were added into the component A, and the mixture was stirred and mixed evenly, so that the sea salt scrub cream was obtained.

Performance test: The test results are shown in Tables 13-14 below.

**Table 13 Relevant performance test results of the Sea salt scrub cream prepared in Application Examples B1-B8**

| | Viscosity at 25°C | Paste spreadability at 25°C | High temperature investigation | Low temperature investigation |
|---|---|---|---|---|
| Application Example B 1 | ○ | ○ | ○ | ○ |
| Application Example B2 | ○ | ○ | ○ | ○ |
| Application Example B3 | ○ | ○ | ○ | ○ |
| Application Example B4 | ○ | ○ | ○ | ○ |
| Application Example B5 | ○ | ○ | ○ | ○ |
| Application Example B6 | ○ | ○ | ○ | ○ |
| Application Example B7 | ○ | ○ | ○ | ○ |
| Application Example B8 | ○ | ○ | ○ | ○ |

**Table 14 Relevant performance test results of the Sea salt scrub cream prepared in Application Comparative Examples B1-B4**

| | Viscosity at 25°C | Paste spreadability at 25°C | High temperature investigation | Low temperature investigation |
|---|---|---|---|---|
| Application Comparative Example B1 | ○ | × | × | × |
| Application Comparative | ○ | × | × | × |
| Example B2 | | | | |
| Application Comparative Example B3 | × | ○ | × | ○ |
| Application Comparative Example B4 | × | ○ | × | ○ |

According to the descriptions in the above Tables 13-14, it can be seen that if alkanoyl taurine salt is used alone as the main surfactant for the sea salt scrub cream, the sea salt scrub cream has a suitable viscosity, but its paste has a strong micelle like texture and is not easy to spread, making it more laborious in actual use. Furthermore, after the high and low temperature investigations, it is easy to cause the paste to become rough. However, if methyl alkanoyl taurine salt is used alone as the main surfactant for the sea salt scrub cream, the sea salt scrub cream has a low viscosity. Although it is easy to spread, the high temperature investigation leaves water and makes it have no texture during use. After using a combination of alkanoyl taurine salt and methyl alkanoyl taurine salt, the advantages thereof can be combined simultaneously, and thus the sea salt scrub cream has an appropriate viscosity, with soft and easy to spread paste, and is good in high and low temperature stability.

The foregoing descriptions are merely exemplary embodiments of the present disclosure. It should be pointed out that for those of ordinary skill in the art, some variations or equivalent substitutions without departing from the principle of the present disclosure can also be made to the technical solutions, and these modifications or equivalent substitutions shall also be regarded as the protection scope of the present disclosure.

## Claims

1. A cleaning composition containing a surfactant system, **characterized in that**, comprising the following components:
A) alkanoyl taurine salt, the structural formula of the alkanoyl taurine salt being as shown in formula 1 below: 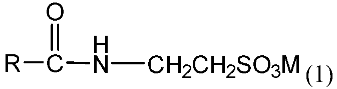 wherein R is a saturated or unsaturated hydrocarbon group of C7-C21, the saturated or unsaturated hydrocarbon group contains branched or unbranched chains, and M is an alkali metal ion or an alkaline earth metal ion; and
B) methyl alkanoyl taurine salt, the structural formula of the methyl alkanoyl taurine salt being as shown in formula 2 below: 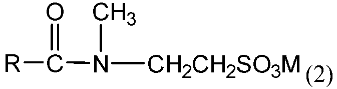 wherein R is a saturated or unsaturated hydrocarbon group of C7-C21, the saturated or unsaturated hydrocarbon group contains branched or unbranched chains, and M is an alkali metal ion or an alkaline earth metal ion.

2. The cleaning composition according to claim 1, **characterized in that**, the C7-C21 are single carbon chains or mixed carbon chains.

3. The cleaning composition according to claim 2, **characterized in that**, each of the C7-C21 is one of lauryl, myristyl or cocoyl.

4. The cleaning composition according to claim 1, **characterized in that**, the alkali metal ion or alkaline earth metal ion is selected from one of sodium, potassium, magnesium, and calcium.

5. The cleaning composition according to claim 4, **characterized in that**, the alkali metal ion or alkaline earth metal ion is selected from one of sodium or potassium.

6. The cleaning composition according to claim 1, **characterized in that**, the total amount of A and B in the cleaning composition is not less than 0.5wt%.

7. The cleaning composition according to claim 6, **characterized in that**, the total amount of A and B in the cleaning composition is 3-30wt%.

8. The cleaning composition according to claim 1, **characterized in that**, the weight ratio of A and B in the cleaning composition is within a range of 10:1 to 1:10.

9. The cleaning composition according to claim 8, **characterized in that**, the weight ratio of A and B in the cleaning composition is within a range of 7:3 to 4:6.

10. The cleaning composition according to claim 1, **characterized in that**, the cleaning composition further comprises a surfactant C besides A and B; and the surfactant C is selected from one or more of anionic surfactants, cationic conditioning agents, non-ionic surfactants, and zwitterionic surfactants.

11. The cleaning composition according to claim 1, **characterized in that**, the cleaning composition further comprises at least one of high-molecular polymers, hydrophobic skin or hair benefiting agents, water-soluble skin or hair benefiting agents, exfoliants, and additives, wherein the additives are selected from at least one of preservatives, chelating agents, antimicrobial agents, fillers, penetrating agents, opacifying agents, fragrances, dispersing agents, and film forming agents.

12. Use of the cleaning composition according to any one of claims 1-11 in the preparation of personal care cleaners.

13. The use according to claim 12, **characterized in that**, the personal care cleaners comprise one or more of shampoo, shower gel, scrub cream and facial cleanser.
